# EUROPEAN PATENT APPLICATION

(11) **EP 1 529 533 A1**
(43) Date of publication of application: **11.05.2005**
(21) Application number: 03025603.6
(22) Date of filing: 06.11.2003
(51) Int. Cl.: A61K 38/25, A61P 9/10

(54) **Use of GH secretagogues in hypoxic-ischemic brain injury**

(71) Applicant: Sahltech I Göteborg AB, 41345 Göteborg (SE)
(72) Inventor: Isgaard, Jörgen, 412 52 Göteborg (SE); Hagberg, Henrik, 435 38 Mölnlycke (SE); Brywe, Katarina Gustafson, 429 42 Särö (SE)
(74) Representative: Becker Kurig Straus

(57) **Abstract**

The present invention relates to the use of a GH secretagogue for treating and/or preventing hypoxia-ischemia brain injury. The instant invention in particular pertains to the use of hexarelin and/or ghrelin for preventing infarction or reducing the infarct volume of the brain.

## Description

The present invention relates to the use of Growth hormone secretagogues for treating and/or preventing hypoxia-ischemia brain injury. Specifically, the instant invention pertains to the use of hexarelin and/or ghrelin for preventing infarction or reducing the infarct volume in vessels of the brain.

Ischemia is generically considered to be a deficiency of blood flow to a particular part of the body, which is mainly caused by constriction or obstruction of a blood vessel, resulting in the cells prominent in said part to be under low or non oxygen, i.e. hypoxic, conditions. Due to a reduced blood flow, loss of physiological homeostasis may lead to ischemic infarction and eventually to cellular death via apoptosis.

Different physiological mechanisms may underlay hypoxic-ischemic injury such as decreased blood flow autoregulation, altered metabolism, thrombosis, hemorrhage, accumulation of toxic metabolites, impaired intracellular calcium turnover, release of interleukines and prostaglandins, iron accumulations and overproduction of free radicals (Amato et al., Europ. J. Paediatric Neurology **4** (2000), 203-209). In the perinatal period hypoxia-ischemia (HI) damage of the brain may even produce irreversible tissue injury resulting in mental retardation, cortical visual deficits and epileptic seizures, and thus contributes to neonatal mortality.

On the molecular level, ischemic-hypoxic injuries involves apoptosis which affects diverse biochemical signalling pathways. Caspase-dependent signalling pathways have been shown to be activated after HI damage in the neonatal rat brain while neuroprotection by BDNF (brain derived neurotrophic factor) blocks this activation. Growth factors, including IGF-1 (insulin-like growth factor-1) a major survival factor in serum, are able to protect cells from apoptosis under a wide variety of circumstances in vitro. IGF-1 anti-apoptotic effects in vitro have been shown to act through activation of phosphatidylinositol 3-kinase (PI3-kinase). It has been demonstrated that PI3-kinase and its downstream kinase Akt also mediate growth factor induced neuronal survival. This signalling pathway starting with PI3-kinase leads to activation through phosphorylation of Akt. P-Akt promotes cell survival and inhibits apoptosis by inactivating several targets including Bad, glycogen synthase kinase-3β(GSK3β ), caspase 9 or FKHLR. Temporal increase of P-Akt levels has been reported after ischemia in different cerebral ischemic models. Another kinase pathway activated by growth factors and known to inhibit apoptosis is the MAPK p42/44 ERK pathway. Activation of ERK has been shown to inhibit apoptosis induced by hypoxia, growth factor withdrawal, H₂O₂ and chemotherapeutic agents. BDNF neuroprotection in neonatal rat proved to be mediated through activation of MAPK/ERK pathway.

Several different intervention therapies have been suggested, including pharmacological and non-pharmacological treatment. Examples are as lowering the temperature of the affected tissue (Dietrich et al., Cellular and molecular mechanism of ischemic brain damage, Siesjo B., Wieloch T., eds., 177-198. Philadelphia: Lippincott-Raven), administering calcium channel blocker (Siesjo et al., Ann. Thorac. Surg. **59** (1995), 1316-1320), or providing free radical scavengers (Chan P., Brain Pathol. (1994), 59-65). An antiischemic and antihypoxic preparation termed "Energostim" contains 0,5 mg of nicotinamide adenine dinucleotide (NAD), 10 mg of cytochrome C and 80 mg of inosine which is the metabolic product of degradation of adenyl nucleotides.

In addition some growth factors and neurotrophins including growth hormone (GH) have been shown to exert neuroprotective activity in animal models, whereas the underlying mechanisms for such protection is not yet fully understood. Apart from growth hormone itself, a growth hormone secretagogue, i.e. Ghrelin, has been shown to possess protective properties for heart tissue and to inhibit apoptosis in cardiomyocytes and endothelial cells through activation of mitogen activated protein kinase (MAPK) and Akt-kinase. Ghrelin, a peptide of 28 amino acids is known to exert its endocrine activity through the Growth Hormone Secretagogue Receptor-1a (GHSR-1a) stimulating GH release.

Yet, in spite of the variety or regimens no satisfactory agent is available at present, with which hypoxic-ischemic injuries may satisfactorily be remedied.

Accordingly, a problem of the present invention is to provide means for ameliorating or preventing effects of conditions of low or no oxygen supply on tissues.

In the course of the extensive studies leading to the present invention it has surprisingly been noted that GH secretagogues, such as hexarelin and ghrelin, also exerts a protective effect on brain tissue exposed to conditions of low or no oxygen supply.

Therefore, the problem has been solved by the use of GH secretagogues or a derivative or a salt thereof for the preparation of a medicament for the prevention and/or treatment of hypoxia-ischemia mediated brain injury.

According to a preferred embodiment, the GH secretagogue is hexarelin and/or ghrelin.

In the figures,
Fig. 1 Percentage tissue loss on four anterioposterior levels of the brain, comparing the left damaged to the right undamaged hemisphere in Hexarelin and vehicle treated animals.
Fig. 2 Neuropathological scoring for the different brain regions and a total scoring for all regions.
Fig. 3 Caspase 3-like activity after Hexarelin or vehicle treatment.
Fig. 4 P-Akt after Hexarelin or Vehicle treatment in hippocampus, cortex and diencephalon
Fig. 5 Phosphorylated GSK3β in the cytosol after hexarelin or vehicle treatment.
Fig. 6 phosphorylated GSK3β in the nucleus after Hexarelin or vehicle treatment.
Fig. 7 Phosphorylated Erk in the nuclear fraction after Hexarelin or vehicle treatment.
Fig. 8 Phosphorylated Akt after HI and Hexarelin or vehicle treatment.

Hexarelin, as an example of a GH secretagogue, is a synthetic hexapeptide capable of stimulating release of growth hormone through the GHSR-1a. It has previously been shown that hexarelin appears to exert beneficial effects in diseases like osteoporosis, obesity and congestive heart failure, with the precise mechanism yet to be elucidated. This spectrum of activities indicated hexarelin to have properties independent of its neuroendocrine activity. During the extensive studies in the field of hypoxic-ischemic brain injuries it has been surprisingly found that hexarelin provides neuroprotective activity. This was demonstrated in an experimental animal model, in which unilateral carotide occlusion provided the insult for only one hemisphere resulting in ischemia followed by hypoxia and tissue infarction in 7 day rats. Administration of hexarelin (5µg) reduced overall brain injury as analysed by area measurements calculating infarction area. Hexarelin alleviated the damage (the infarct volume) especially in three regions of the brain namely the cortex, hippocampus and thalamus (diencephalon).

Equally remarkable, establishment of a neurological scoring (a qualitative as well as quantitative measure of a tissue sample) assessing severity of cell damage revealed that hexarelin compared to vehicle treated brains (brain tissues) showed a significant lower scoring of said specific brain areas, indicating a protective activity. Additionally in the diencephalon 24h after insult and hexarelin injection biochemical analysis showed that caspase activity was reduced indicating that hexarelin acted via anti-apoptosis associated pathways in some regions of the brain.

Accordingly, as hexarelin obviously seems to lead to a reduction of infarct volume of the injured brain area, hexarelin may in fact prevent hypoxia-ischemia mediated brain damage, when applied before or even after an ischemic event, which may prove in particular beneficial when giving birth to a child, avoiding deleterious consequences of low or non-oxygen conditions potentially occurring in perinatal/neonatal periods. Since hexarelin acts along endogenous physiological pathways, undesirable side effects may be avoided by virtue of intact negative feedback loops.

According to a preferred embodiment hypoxic-ischemic brain injuries to be treated accordingly are selected from consisting of stroke, or other inflammatory or degenerative conditions associated with hypoxic ischemic brain injury.

According to yet another embodiment the GH secretagogue, specifically hexarelin and/or ghrelin, provides neuroprotective activity. Generally, the mammalian brain is structured in five segments, which develop from five brain bubbles of the embryo and form the myelencephalon, metencephalon, mesencephalon, diencephalon with the thalamus and hypothalamus, and the prosencephalon with its hemispheres, cortex, hippocampus and striatum. In the context of the present invention, the term "diencephalon" will be used interchangeably with the term "thalamus" in the present application. Neuroprotection means that in all of the five segments the infarct volume of the respective injured brain area may be reduced by the adminstration of the GH-secretagogue, which is performed by anti-apoptotic activity.

The GH secretagogue, i.e. hexarelin and/or ghrelin, will be administered to an individual in need there of in a pharmaceutically sufficient amount. A pharmaceutically sufficient amount shall designate a quantity, sufficient to achieve a desired therapeutical outcome. Such an amount typically ranges of from about about 0,002 to about 200 mg/kg body weight daily and may vary depending on whether the GH-secretagogue is the sole active agent, or the mode or route of administration and the duration of treatment. A preferred regimen ranges of from about 0,02 to about 20 mg/kg body weight daily, more preferably from about 0,2 to about 5 mg/kg body weight daily. An effective amount of 0,02 to 20 mg/kg of body weight daily can be administered as a single dose.

The route of administration may be any route which will effectively transport the active agent/agents to the appropriate tissue or desired area of action. Routes such as oral, nasal, pulmonary or parenteral (subcutaneous, intramuscular, intraperitoneal, intravenous, intracranial injection) may be suitable, while nasal or pulmonary routes being preferred.

For oral administration the composition to be used according to the present invention may be in form of e.g. tablets, troches, lozenges, aqueous or oily suspensions, dispersible powders or granules, emulsions, hard or soft capsules, or syrups or elixirs.

Compositions intended for oral use may be prepared according to any method known in the art for the manufacture of pharmaceutical compositions and such compositions may contain one or more agents selected from the group consisting of sweetening agents, flavoring agents, coloring agents and preserving agents in order to provide pharmaceutically elegant and palatable preparations.

Tablets contain the active ingredient(s) in admixture with non-toxic pharmaceutically acceptable excipients, such as inert diluents, granulating, disintegrating and lubricating agents, which are suitable for the manufacture of tablets. The tablets may be uncoated or they may be coated by known techniques to delay disintegration and absorption in the gastrointestinal tract and thereby provide a sustained action over a longer period.

Formulations for oral use may also be presented as hard gelatin capsules wherein the active ingredient is mixed with an inert solid diluent, or as soft gelatin capsules wherein the active ingredients is mixed with water or an oil medium.

Aqueous suspensions contain the active material in admixture with excipients suitable for the manufacture of aqueous suspensions, such as e.g. suspending agents, dispersing or wetting agents, preservatives, coloring agents, flavoring agents, and sweetening agents.

Dispersible powders and granules suitable for preparation of an aqueous suspension by the addition of water provide the active ingredient(s) in admixture with a dispersing or wetting agent, suspending agent and one or more preservatives. Additional excipients, for example sweetening, flavoring and coloring agents, may also be present.

The GH secretagogue may also be administered in form of an injectable solution (intramuscular, intraperitoneal, intravenous, intracranial injection).

Apart from that, the nasal or pulmonary routes are preferred, since it convey the active ingredient directly to the desired area.

The GH secretagogue will be contained in a pharmaceutically acceptable carrier or diluent, which will be selected by the skilled person according to the route of administration. The pharmaceutical carrier or diluent employed may be a conventional solid or liquid carrier, such as water.

The pharmaceutical composition to be used may comprise the GH secretagogue and at least one additional neuroprotective agent. Such agents may be selected from the group consisting of peptide and/or non peptide components/compounds that exert the same or similar biological effect as the specific GH-secretagogue used in vivo/at the site of action. The at least one additional neuroprotective agent may be selected from e.g. the growth hormone releasing peptide-6 (GHRP-6), GHRP-1, growth hormone releasing factor (GRF), IGF-1, IGF-2, while non-peptide agents may be calcium channel blockers, anti-oxidative stress associated agents.

The GH secretagogue used according to the present invention may be the compound itself, i.e. hexarelin and/or ghrelin, or a derivative thereof or a salt thereof or mixtures thereof. As a derivative of the GH secretagogue the (poly)peptide comprising amino acids in the L- or D-form amino acids are considered, or the (poly)peptide, to which a protective moiety has been attached. Preferably the GH-secretagogue, e.g. hexarelin and/or ghrelin, is in its native form. The GH-secretagogue may also be provided in form of a pharmaceutically acceptable salt.

The following examples illustrate the invention without limiting it thereto.

### Examples:

### Abbreviations used:

- BSA: Bovine serum albumin
- CHAPS: 3-[(3-Cholamidopropyl)-dimethylamminio]-1-propansulfonate
- DTT: Dithiothreitol
- DAB: 3,3'-Diaminobencindine
- EDTA: Ethylenediaminetetraacetate
- EGTA: Ethylen Glycol-bis (β-aminoethyl Ether) tetraacetate
- IGF-1R: Insulin like growth factor-1 receptor
- PBS: Phosphate-buffered saline
- PMSF: Phenylmethylsulfonylfluoride
- SDS-PAGE: Sodiumdodecylsulfate-polyacrylamide-gel-electrophoreses
- Tris: Tris(hydroxymethyl)aminomethane

### Materials and Methods

### Animal model

In a neonatal model that has previously been described (Rice J. E., V. R. C. Brierley, Ann. Neurol. **9** (1981), 1126-35; Hagberg H. et al., Acta Paedeatr. Suppl **442** (1997), 85-88). Seven-day-old out bred Wistar Fue rats were anesthetised with enflurane (3,5% for induction and 1,5% for maintenance) in a mixture of nitrous oxide and oxygen on a snout mask. The left common carotid artery was cut between two prolene sutures (9-0). After anesthesia and surgery the animals were returned to there dams and allowed to recover for 60 minutes. They were thereafter exposed to 60 minutes of hypoxia in a humidified chamber at 36°C with 7.7±0.01% oxygen in nitrogen. The pups were returned to and kept with their dams until they were killed at different time points after the insult Animals were either perfused intracardially with saline and 5% buffered formaldehyde (Histofix; Histolab, Göteborg, Sweden) for preparation of paraffin sections or fresh tissue were immediately frozen in isopenthane on dry ice for preparation of protein homogenates. The animal ethics committee in Gothenburg approved the animal studies.

### Intracerebral injection of hexarelin and vehicle

Animals received daily intracerebral injections of 5 µl solution containing either 5µg hexarelin or vehicle (NaCl 0.9 mg/ml). Injections were headed for the left ventricular at 1.5 mm lateral to the Sagittal suture, 5.7 mm rostral to lambda and 2.8 mm deep to the skull surface with a special made needle of 27 gauge. Injection velocity of 1.5 µl/min with a Hamilton syringe connected to a MCA pump.

### Western blot

Hexarelin treated rats were decapitated at 30 minutes, 2hr, 8hr and 24hr, vehicle treated animals at 2h after injection. Rats exposed for HI and Hexarelin treatment were decapitated at 8hr after insult and injection. Brains were rapidly harvested, hemispheres were split and hippocampus, cortex, thalamus and striatum dissected out. Each region was immediately homogenised in ice-cold homogenisation buffer (50mM Tris, pH 7.3 containing 5 mM EDTA) and protease and phosphatase inhibitor cocktail were added to a final concentration of 1% and 2% respectively. The homogenates were spun at 800g 4°C for 10 minutes for preparation of nuclear fraction. Supernatants were spun at 9200g 4°C for 15 minutes for preparation of cytosolic fraction. Both nuclear and cytosolic fraction were used for Western Blot analysis. Protein content was quantified using the method presented by Whitaker and Granum (Anal. Biochem. 109 (1980), 156-159) adapted for microplates. Samples were mixed with an equal volume of 3X SDS-PAGE buffer and heated (96°C) for 5 min. Samples were electrophoresed on Novex (San Diego, CA) precast 8-16% Tris-glycine gels and transferred to nitrocellulose (Optitran, 0,2um; Schleicher & Schuell, Dassel, Germany) membranes. Membranes were blocked in 30mM Tris-HCl, pH 7,5 100mM NaCl and 0,1% Tween 20 (TBS-T) containing 5% fat free milk powder. Incubation with the following primary antibodies diluted in TBS-T containing 3% BSA and 9 mM NaN₃ for 1 hr in room temperature; anti-phospho-Akt (Ser⁴⁷³) rabbit polyclonal 1:1000, anti-Akt rabbit polyclonal 1:1000, anti-phospho-GSK3β (Ser9) rabbit polyclonal 1:1000, anti-GSK3 mouse monoclonal 1:500, Cell Signalling Technology, Beverly, MA, USA anti-phospho-p44/42 MAP-kinase rabbit polyclonal 1:1000, anti-p42/44 MAP-kinase rabbit polyclonal 1:1000, Cell Signalling Technology, Beverly, MA, USA. Blots were washed 3 times with TBS-T and incubated with the appropriate secondary antibodies peroxidase conjugated (Vector Laboratories, Burlingame, CA) diluted in blocking buffer. Immunoreactive species were visualised using Super Signal Western Dura chemiluminescence substrates (Pierce Rockford, IL) and a cooled CCD camera (LAS1000; Fujifilm, Tokyo, Japan). Immunoreactive bands were quantified using Image Gauge software (version 3.3; Fujifilm). On every gel the same five controls were run to standardise the measurements. Every sample was analysed three times and the average value was used as *n=1.* Membranes were stripped for re-probing with new antibodies. Membranes were incubated in stripping buffer (62.5mM Tris-HCl, pH 6.7, 100mM β-mercaptoethanol and 2% SDS at 45°C) for 30 minutes.

### Evaluation of brain damage

Animals were perfused at 72hr *(n=27* hexarelin treated HI animals and *n=27* vehicle treated HI animals) brains were removed from the skull and immersion fixed at 4 °C for 24hr, dehydrated, embedded in paraffin and cut into 5µm-thick coronal sections on four anteroposterior levels from the anterior striatum to the posterior part of hippocampus. Adjacent sections were stained with thionin/ acid fuschin (Mallard et al., Pediatr Res. 33 (1993), 61-65) and for microtubule associated protein MAP-2 (1:1000, 1hr, mouse anti MAP-2, clone HM-2;Sigma) immunoreactivity was visualised with DAB as described above.

### A. Neuropathological scoring

Brain injury was evaluated and observer blinded between the study groups using a neuropathological scoring system where injury in cortex was graded from 0-4 with 0 being no observable injury and 4 being confluent infarction encompassing most of the hemisphere. The damage in the hippocampus, thalamus and striatum was assessed regarding both hypothrophy (shrinkage of cells) 0-3 and observable cell injury/infarction 0-3, resulting in a scoring for each region 0-6. The total score 0-22 was the sum score for all the four regions.
The rectal temperature which has been shown to correspond to the brain core temperature (Thoresen M. et al., Arch. of disease in Childhood 74 (1996), F3-F9) were measured in two litters at 1h, 3h, 6h, 12h, 24h, 36h, 48h and 72h after HI.

### B. Area measurement

Intact neurons (dendrites and soma) express MAP-2 and infarction in gray matter is associated with a distinct loss of MAP-2 immunoreactivity. MAP-2 positive areas in the ipsi- and contralateral hemispheres where outlined by an observer blinded to the study groups and where then calculated using the Olympus Micro Image, image analysis software version 4.0 (Olympus Optical, Tokyo, Japan). The proportion of the infarction was calculated by subtracting the MAP-2 positive area of the ipsilateral hemisphere from the contralateral hemisphere and was expressed as percentage of the contralateral hemisphere.

### Statistics

The data were expressed as means ± S.E.M. The tests used were Mann Whitney non parametric test and ANOVA. Values of p<0.05 were considered to be significant. In all cases n correspond to the number of animals.

### Fluorometric assay of caspase-3 like activity

Tissue samples from cortex, diencephalon and hippocampus were dissected and immediately frozen in isopenetane on dry ice at 24 hours after HI and hexarelin treatment in both lesioned and unlesioned hemispheres. The tissue samples were homogenized by sonication in ice-cold homogenization buffer (50mM TRIS, pH 7.3 containing 5mM EDTA). Samples were aliquoted and stored at -80°C. The protein concentration was determined according to the method presented by Whitaker and Granum (Whitaker J. G. et al., Anal. Biochem. 109 (1980), 156-159). Samples of homogenate or cytosolic fraction were mixed with extraction buffer ( 50mM Tris-HCl pH 7.3, 100mM NaCl, 5mM EDTA, 1mM EGTA, 3mM NaN3, 1mM PMSF, 1ug/ml pepstatin, 2.5ug/ml leupeptin, 10ug/ml aprotinin 0.2% CHAPS, protease inhibitor cocktail (P8340; Sigma, St Louise, MO) 1%) 1:3, on a micro-titerplate. (Microflour, Dynatech, Ca, USA). After incubation for 15min at room temperature, 100ul of peptide substrate, 50uM Ac-Asp-Glu-Val-Asp-aminomethyl coumarine (Ac-DEVD-AMC; Enzyme systems Products, Livermore, CA, USA) in extraction buffer without inhibitors or CHAPS, but with 4mM DTT added. Cleavage of the substrate was measured at room temperature using a Perkin-Elmer LS 50B luminescence spectrometer with a microtiter plate attachment, at an excitation wavelength of 380nm (slit 15nm) and emission wavelength of 460nm (slit 20nm). The degradation was followed at 2-min intervals for 1-2h, and V-max was calculated from entire linear part of the curve. Standard curves with AMC in the appropriate buffer were used to express the data in pmoles of AMC formed per minute and milligram of protein.

### RESULTS

### Brain injury in Hexarelin and Vehicle treated animals

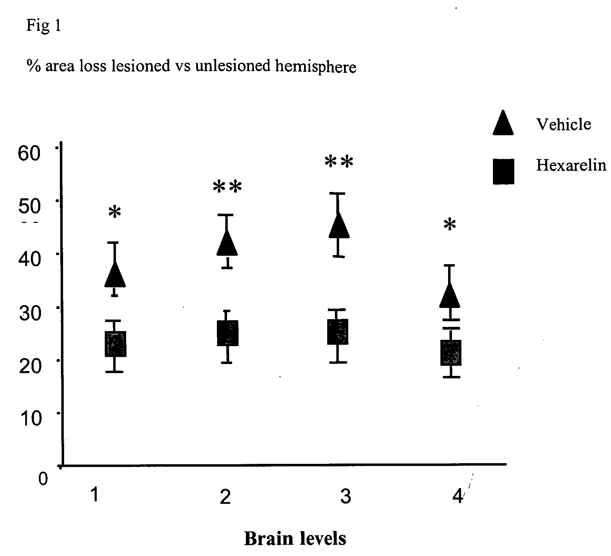

Infarcted area measured as loss of MAP-2 staining in the damaged ipsilateral hemisphere compared to the contralateral hemisphere were significantly reduced on all four levels of the brains studied in the Hexarelin treated group compared to the control group (Fig. 1). Neuroprotection was most pronounced at level 3 over hippocampus (-2 mm from bregma) with a 43% reduction (from 45,2% ± 4,6 to 25,7% ± 3,5, p<0,01) in infarcted tissue, this is also the level where the damage is largest in this animal model. A mean of the four levels shows a reduction in the treatment group by 34% (from 36.4,% ± 3,5 to 24.0% ± 2,9 p<0.01).

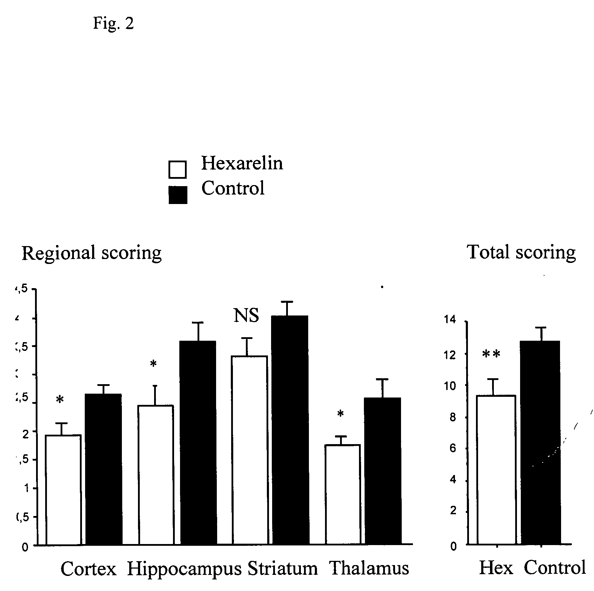

The morphological analysis showed an overall reduced brain injury in hexarelin compared with vehicle treated animals. The neuropathological score of the whole brain was significantly reduced by 27% (from 12.7 ± 0.9 to 9.3 ± 1.1 p<0.05) in the hexarelin group compared to control. Specific brain regions showed protection: cortex (HEX: 1.9+/- 0.2 vs. VEH: 2.6+/- 0.2, p<0.05), hippocampus (HEX: 2.4±0.3 vs. VEH: 3.6±0.3, p<0.05), thalamus (HEX: 1.7±0.2vs. VEH: 2.6±0.3, p<0.05)(Fig. 2). There was no significant protection in the striatum (p=0.07). There were no differences in body temperature, mortality or body weight (data not shown) between the two groups. Moreover the were no significant differences between male and female animals in the study.

### Caspase 3 activity

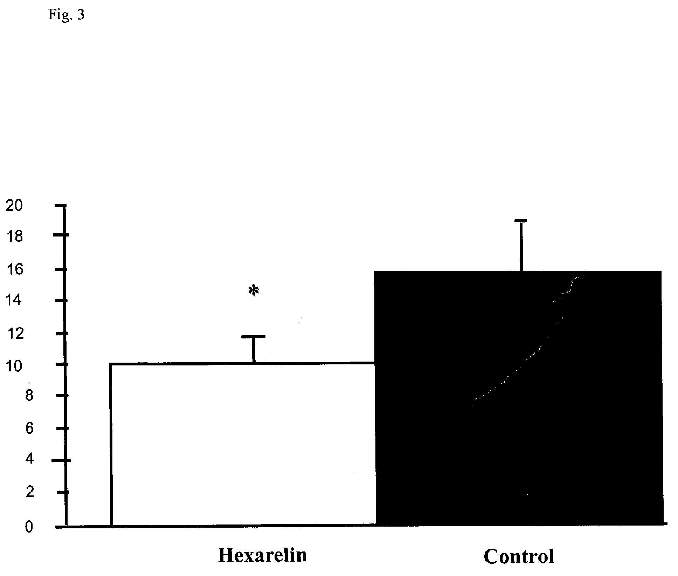

Since Caspase-3 activity has been shown to be increased in the neonatal brain after HI and is believed to play a relatively more important role in the immature brain compared to the adult brain (Han, D'Costa et al., Neurobiol. Dis. **7** (2000), 38-53) it was investigated whether hexarelin neuroprotection has effects on the apoptotic component of brain injury, mediated through inhibition of caspase-3 activity. At postnatal day 7 HI insult were induced followed by intracerebral injection of Hexarelin n=28 or vehicle (saline) n=26. Caspase-3 activity (DEVD cleavage) was measured in the diencephalon and cortex 24h after insult and treatment. Caspase-3 was significantly reduced in the ipsilateral diencephalon of Hexarelin treated animals compared to controls. A reduction by 37% of Caspase-3 activity (from 15,9±3,0 to 10,0±1,9 p<0,05)(Fig. 3). There were no significant differences in cortex at either timepoint.

### Activation of P-Akt pathway after Hexarelin injection i.c.v.

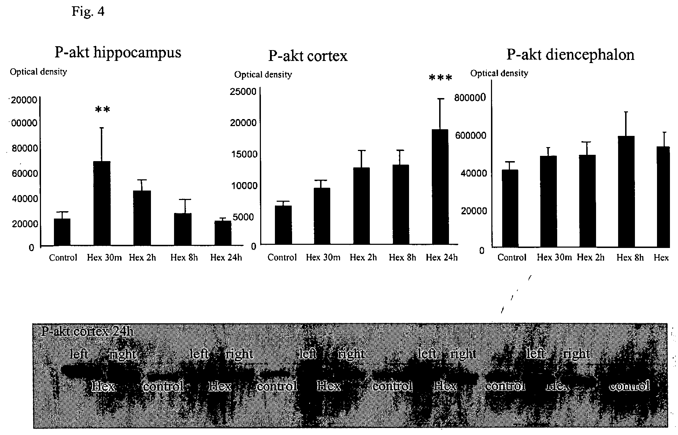

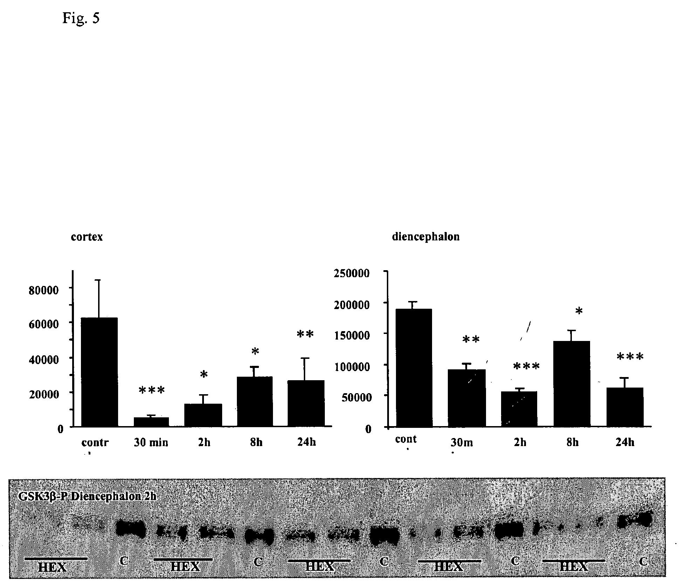

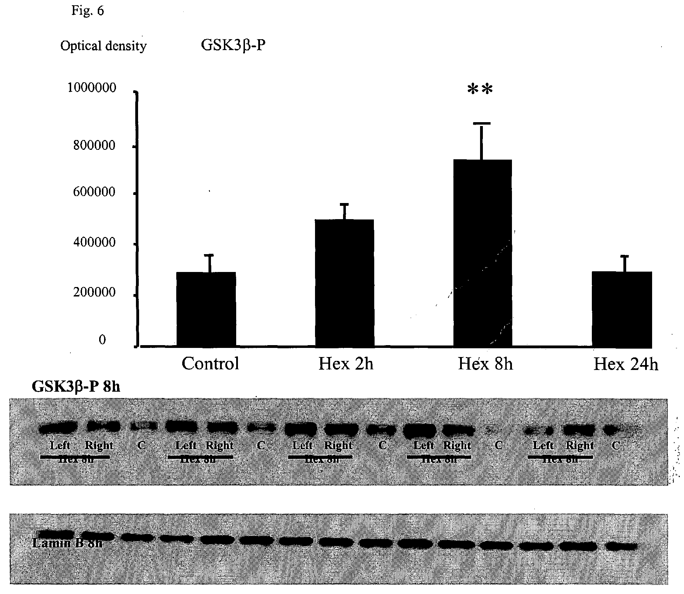

Immunoblots of P-Akt, Akt, P-GSK3β and GSK3 were prepared from cortex, hippocampus and diencephalon cytosolic and nuclear fractions at 30 min, 2hr, 8hr and 24 hr after Hexarelin n=5 or vehicle (saline) n=5 treatment. Activated (phosphorylated) Akt was increased in the cytosol after Hexarelin treatment compared to saline after 30min in hippocampus, 24hr in cortex and there were a similar pattern but no significant differences in the diencephalon (Fig. 4). There were no changes seen in the total Akt levels in any regions. Unexpectedly phosphorylated (inactivated) GSK3β was decreased in the cytosolic fraction of Hexarelin treated animals from 30 min and still at the latest time point 24hr in cortex and diencephalon (Fig. 5). In contrast there was a pattern of increase of the P-GSK3β in the nuclear fraction after 2hr but significant first at 8hr (Fig. 6). There seem to be a sub-cellular difference in P-GSK3p moving from the cytosol to the nucleus after Hexarelin treatment. Total GSK3 did not differ between the treatment groups.

### Activation of MAPK pathway after Hexarelin injection i.c.v.

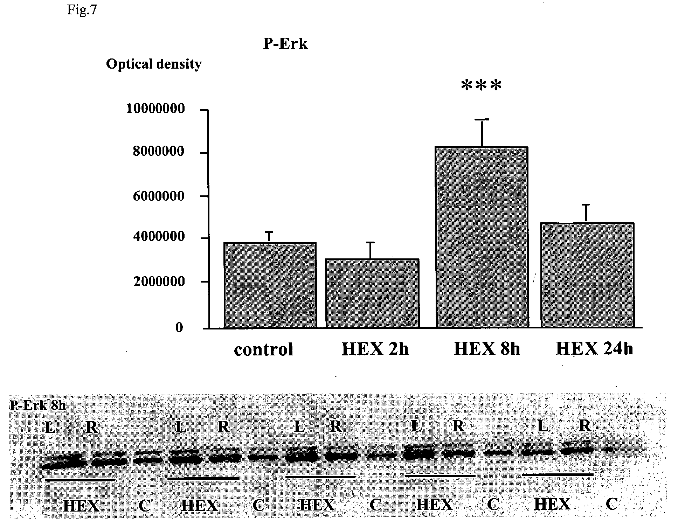

Immunoblots of phospho-p44/42 (p-Erk) and p44/42 (total-Erk) were prepared from nuclear fractions of the whole hemisphere 2hr, 8hr and 24hr after i.c.v. treatment with Hexarelin n=5 and vehicle (saline) n=5. Phosphorylated Erk was increased at 8hr after Hexarelin treatment compared to vehicle (1074569±59997 vs. 521452±86006, densitometry p<0.0001), whereas there were no differences in the total Erk between the two groups (Fig 7).

### Activation of P-Akt after Hexarelin treatment following Hypoxia-Ischemia

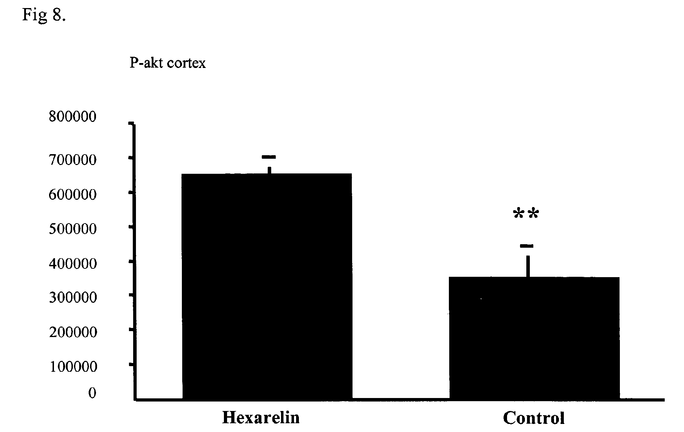

Preparation of immunoblots 24hr after HI insult and Hexarelin n=7 or vehicle n=7 treatment also shows a increase in phosphorylated Akt in the cortex and diencephalon of cytosolic fraction (Fig. 8). There were no differences in total Akt or P-GSK3β at the studied time point.

## Claims

1. Use of a GH-secretagogue or a derivative or a salt thereof for the preparation of a medicament for the treatment and/or prevention of hypoxic-ischemic brain injury.

2. The use according to claim 1, wherein the GH-secretagogue is hexarelin or ghrelin.

3. The use according to claim 1 or 2, wherein hypoxic-ischemic brain injury is selected from the group consisting neonatal hypoxic ischemic brain injury, stroke or other inflammatory or degenerative conditions associated with hypoxic ischemic brain injury.

4. The use according to any of the preceding claims, wherein the GH-secretagogue provides neuroprotective activity.

5. The use according to any of the preceding claims, wherein neuroprotection comprises reduction in infarct volume, preferably via anti-apoptotic activity.

6. The use according to any of the preceding claims, wherein the GH secretagogue is administered in a pharmaceutically sufficient amount.

7. The use according to claim 6, wherein the amount administered is from about 0,2 mg/kg to 200 mg/kg, preferably from about 0,5 mg/kg to 20 mg/kg,

8. The use according to claim 7, wherein the amount administered is from about 0,5 mg/kg to 5 mg/kg body weight.

9. The use according to any of the preceding claims, wherein the GH secretagogue is administered with a pharmaceutical acceptable carrier, diluent or solvent.

10. The use according to any of the preceding claims, wherein the GH secretagogue is administered together with another neuroprotective agent.
